(19) Europäisches Patentamt / European Patent Office / Office européen des brevets

(11) **EP 3 863 582 B1**

(12) **EUROPEAN PATENT SPECIFICATION**

(45) Date of publication and mention
of the grant of the patent:
**17.01.2024 Bulletin 2024/03**

(21) Application number: **19794383.0**

(22) Date of filing: **09.10.2019**

(51) International Patent Classification (IPC):
**A61F 13/515** (2006.01)    **A61F 13/539** (2006.01)
**A61F 13/84** (2006.01)    **D04H 1/587** (2012.01)
**D04H 1/593** (2012.01)    **A61L 15/24** (2006.01)
**A61L 15/42** (2006.01)

(52) Cooperative Patent Classification (CPC):
(C-Sets available)
**D04H 1/587; A61F 13/515; A61F 13/539;
A61F 13/8405; A61L 15/24; A61L 15/42;
D04H 1/593**    (Cont.)

(86) International application number:
**PCT/US2019/055333**

(87) International publication number:
**WO 2020/076907 (16.04.2020 Gazette 2020/16)**

(54) **ABSORBENT ARTICLE WITH A SUBSTANTIALLY TACKIFIER-FREE POLYMERIC FILLER COMPOSITION**

ABSORBIERENDER ARTIKEL MIT EINER IM WESENTLICHEN KLEBRIGMACHERFREIEN POLYMEREN FÜLLSTOFFZUSAMMENSETZUNG

ARTICLE ABSORBANT AVEC UNE COMPOSITION DE CHARGE POLYMÈRE SENSIBLEMENT EXEMPTE D'AGENT POISSEUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.10.2018 US 201862743266 P
17.04.2019 PCT/CN2019/082955**

(43) Date of publication of application:
**18.08.2021 Bulletin 2021/33**

(73) Proprietor: **The Procter & Gamble Company
Cincinnati, OH 45202 (US)**

(72) Inventors:
• **TURNER, Robert, Haines
Cincinnati, Ohio 45202 (US)**
• **STRASEMEIER, John, Andrew
Cincinnati, Ohio 45202 (US)**
• **LINDNER, Torsten
65824 Schwalbach am Taunus (DE)**
• **MORAND, Mathias
65824 Schwalbach am Taunus (DE)**

(74) Representative: **P&G Patent Germany
Procter & Gamble Service GmbH
Sulzbacher Straße 40
65824 Schwalbach am Taunus (DE)**

(56) References cited:
**CN-U- 204 095 212        US-A1- 2016 270 986
US-A1- 2016 270 987        US-A1- 2017 165 130
US-A1- 2017 165 133**

(52) Cooperative Patent Classification (CPC): (Cont.)

C-Sets
**A61L 15/24, C08L 23/12**

C-Sets
**A61L 15/24, C08L 23/12**

**Description**

FIELD

The present application is for absorbent articles, such as diapers, comprising one or more bond area(s) between two nonwovens. A polymer filler composition which is substantially tackifier-free is disposed within the bond area(s). The polymeric filler composition comprises at least one of a polypropylene homopolymer, a propylene ethylene copolymer, or a mixture thereof. The polymeric filler composition is particularly useful to form nonwoven to nonwoven (NW-NW) bonds having high peel creep requirements, such as the bonds between the top and bottom sides of an absorbent core wrap, or the bonds between the landing zone and the backsheet outer cover for taped absorbent articles.

BACKGROUND

Disposable absorbent articles, such as diapers, training pants or adult incontinence articles, are generally manufactured by combining several components. These components typically include a chassis comprising a liquid-permeable topsheet, a liquid-impermeable backsheet attached to the topsheet, an absorbent core located between the topsheet and the backsheet, and a plurality of bond areas holding the chassis together. When the disposable article is worn, the liquid-permeable topsheet is positioned next to the body of the wearer. The topsheet allows passage of bodily fluids into the absorbent core. The liquid-impermeable backsheet helps prevent leakage of fluids held in the absorbent core. The absorbent core typically comprises superabsorbent polymers (SAP) that can absorb several times their weight of urine or other liquid, so that bodily fluids can be transported and stored from the skin of the wearer into the disposable absorbent article.

Frequently one or more components of a disposable absorbent article are bonded together. For example, hotmelt adhesives have been used to bond individual layers of the chassis of the absorbent article, such as the topsheet and backsheet together. Hotmelt adhesives have also been used to bond discrete components, such as fasteners and leg elastics or cuffs, to the article. The hot melt adhesive is often called a construction adhesive because it is used to help construct the absorbent article from individual components.

Common hotmelt adhesives are made by combining polymers and additive components in a substantially uniform thermoplastic blend. Typical additive components include tackifiers, plasticizers, and/or waxes. While such formulations generally work, they can be costly and their performance properties can be improved. Tackifiers for example can comprise up to 65% of an adhesive formula, and can be expensive and difficult to source.

Instead of using formulated adhesives, unblended polymers have been proposed. An unblended polymer consists only of one type of polymer (generated via its own and specific polymerization process) rather than a blend of polymers which were made via separate polymerization process and mixed (blended) together after polymerization. Unblended polymers may additionally comprise minor amount of additives such as antioxidants, perfumes and other low molecular weight components, but is substantially free of other polymers, mineral oils, or tackifiers.

The advantages of unblended polymers typically include higher purity, enabling less odor, at a lower cost as the additional steps of heating up, blending and cooling down a blend can be avoided. Unblended polymers can have higher resistance to degradation of the bond over time because lower molecular weight components cannot migrate into adjacent materials. US2016/0053149A1 (Clariant) for example discloses a ready-to-use hotmelt adhesive comprising at least 95% of one or more polyolefin copolymer waxes, which have been prepared by means of metallocene catalysts, characterized in that the polyolefin copolymer wax consists of propylene and one or more further monomers selected from ethylene and branched or unbranched 1-alkenes having 4 to 20 C-atoms and the content of structural units derived from propylene in the copolymer waxes amounts to 80 to 99.9% by weight, and the hot melt adhesive has a surface tension of the melt, measured at a temperature of 170 °C, of at most 23 mN/m.

Examples of various adhesive compositions used for bonding absorbent article components together are disclosed in US2016/0270987 A1, US2016/0270986 A1, US2017/0165130 A1 and US2017/0165133 A1.

Some bonds in diapers need to withstand demanding requirements related to "peel creep", i.e. exposure of a sustained peeling force over an extended period of time, typically at body temperature. For example, the end seal bonds of core bag should be able to withstand the forces of the swelling SAP in use. Another example is the landing zone material that is attached to the front side of taped diapers and on which the back ears can be releasably fastened. The peel creep resistance can be measured via static hang tests. It was found that a load applied in the "Peel" configuration is more damaging to bonds than a load applied in the "Shear" configuration. It was also found that the required properties of adhesives for NW-NW bonds are very different than for NW-Film bonds.

Despite their advantages, polyolefin (PO) adhesives are typically viscoplastic and prone to show creep in static tests. This limits significantly their use for such bonds that require creep resistance, and in cases where they are not completely unsuitable requires high usages.

There is thus a need for a polymeric material, referred herein to as polymer filler composition, that is substantially tackifier-free and at the same time efficient to bond two nonwoven materials, especially when these bonds are subjected to peel creep force in an absorbent article.

SUMMARY

The present invention is for an absorbent article comprising a first nonwoven material joined to a second nonwoven material by at least one bond area, wherein a polymeric filler composition is disposed within the bond area and the composition comprises at least one polymer selected from the group consisting of polypropylene homopolymers, propylene-ethylene copolymers, and mixtures thereof, wherein the composition is substantially free of tackifiers, comprising less than 5 % of tackifiers, by weight of the polymeric filler composition. It was found that such polymer filler compositions having a Toughness of at least 25 MPa, as measured according to the Extensional Test Method described herein, provide bonds with good resistance to peel creep force.

BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:

Fig. 1 shows a perspective view of an exemplary taped diaper in a closed configuration as it would be when worn by a wearer;
Fig. 2 shows the garment-facing side of the diaper of Fig. 1 with the diaper flattened out;
Fig. 3 shows the wearer-facing side of the diaper of Fig. 1 with the diaper flattened out;
Fig. 4 shows a top view of an exemplary absorbent core with the top layer partially removed;
Fig. 5 shows a longitudinal cross-section view of the absorbent core of Fig. 4;
Fig. 6 shows transversal cross-section view of the absorbent core of Fig. 4;
Fig. 7 is a schematic sketch showing Shear load and Peel load applied on a bond area;
Fig. 8 is SEM of a representative bond area.

DETAILED DESCRIPTION

Introduction

"Absorbent article", as used herein, refers to devices that are placed against or in proximity to the body of the wearer to absorb and contain the various exudates discharged from the body. Absorbent articles may include baby diapers, training pants, adult incontinence undergarments, feminine hygiene products, and the like. As used herein, the term "body fluids" or "body exudates" includes, but is not limited to, urine, blood, vaginal discharges and fecal matter.
"Absorbent core" means an absorbent structure disposed between topsheet and backsheet for absorbing and containing liquid such as urine received by the absorbent article. The absorbent core comprises an absorbent material, that is typically enclosed within or sandwiched between a core wrap. The core wrap may be a single material that is folded and attached to itself, or it may comprise a separate top layer and bottom layer that are bonded together. The absorbent material typically comprises superabsorbent particles which are optionally mixed with cellulose fibers. As used herein, "absorbent core" does not include any acquisition-distribution systems, topsheet, or backsheet of the absorbent article. The absorbent core may consist essentially of the core wrap, the superabsorbent polymer particles, and the immobilizing material as a fibrous network.
"Amorphous" refers herein to the substantial absence of crystallinity, in particular to polymers having an enthalpy of fusion of less than 20.0 J/g, in particular from 0 to 10 J/g, as measured with the Enthalpy of Fusion Test Method described herein. The amorphous nature of the polyolefin material results in a melting point, which is not sharp or definite. Rather as the temperature increases, amorphous polymers gradually change from a solid to a soft and then to a liquid material. No clearly defined glass transition or melting temperature is often noted.
"Comprise," "comprising," and "comprises", as used herein, are open ended terms, each specifies the presence of what follows, e.g., a component, but does not preclude the presence of other features, e.g., elements, steps, components known in the art, or disclosed herein.
"Consisting essentially of", as used herein, limits the scope of subject matter, such as that in a claim, to the specified materials or steps and those that do not materially affect the basic and novel characteristics of the subject matter.
"Diaper", as used herein, refers to an absorbent article generally worn by infants and incontinent persons about the lower torso so as to encircle the waist and legs of the wearer and that is specifically adapted to receive and contain urinary and fecal waste. As used herein, term "diaper" also includes "pants" which is defined below.

"Nonwoven", as used herein, is a manufactured sheet, web, or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than 0.001 mm to greater than 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as meltblowing, spunbonding, solvent spinning, electrospinning, and carding. The basis weight of nonwoven fabrics is usually expressed in grams per square meter (gsm of g/m$^2$).

"Pant" or "training pant", as used herein, refer to disposable garments having a waist opening and leg openings designed for infant or adult wearers. A pant may be placed in position on the wearer by inserting the wearer's legs into the leg openings and sliding the pant into position about a wearer's lower torso. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened). While the terms "pant" or "pants" are used herein, pants are also commonly referred to as "closed diapers," "prefastened diapers," "pull-on diapers," "training pants," and "diaper-pants".

"Substantially", as used herein, means generally the same or uniform but allowing for or having minor fluctuations from a defined property, definition, etc. For example, small measurable or immeasurable fluctuations in a measured property described herein, such as viscosity, melting point, etc. may result from human error or methodology precision. Other fluctuations are caused by inherent variations in the manufacturing process, thermal history of a formulation, and the like. The compositions of the present invention, nonetheless, would be said to be substantially having the property as reported.

"Superabsorbent polymer" or "SAP" refers herein to absorbent materials, typically crosslinked polymeric materials, that can absorb at least 10 times their weight of an aqueous 0.9% saline solution as measured using the Centrifuge Retention Capacity (CRC) test (EDANA method WSP 241.2.R3 (12)). The SAP may in particular have a CRC value of at least 20 g/g, in particular of from 20 to 40 g/g. "Superabsorbent polymer particles", as used herein, refers to a superabsorbent polymer material which is in particulate form so as to be flowable in the dry state.

General description of an absorbent article

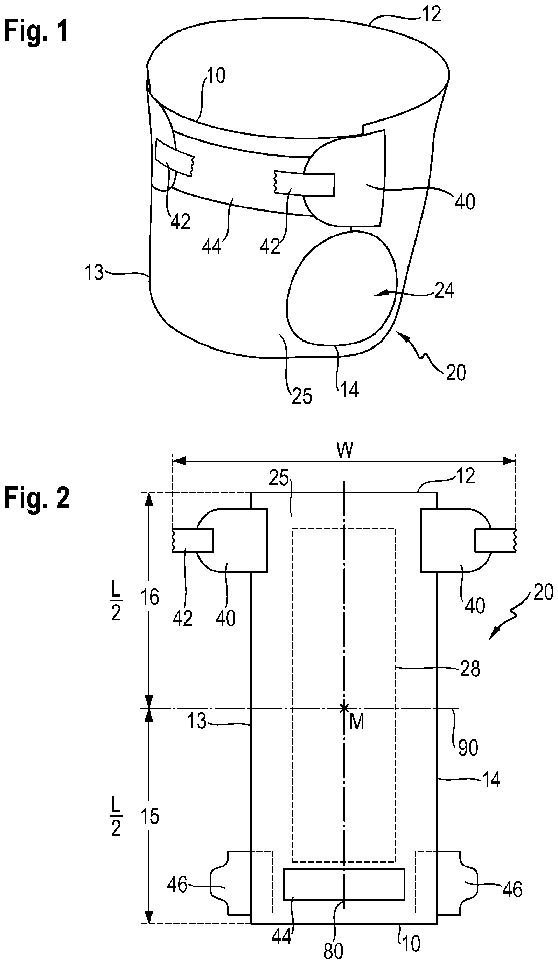

An exemplary absorbent article according to the invention in the form of a baby taped diaper 20 is represented in Figs. 1-3. Fig. 1 is a perspective view of the exemplary diaper in a closed state as it would appear when worn by a wearer. This taped diaper 20 is shown for illustration purpose only as the invention may be used for making a wide variety of diapers or other absorbent articles such as baby diaper pants, adult incontinence pants or feminine sanitary pads. In the following, the word "diaper" and "absorbent article" are used interchangeably. The Figures are used herein as illustration of one way to carry out the invention and are not limiting the scope of the claims, unless specifically indicated to do so.

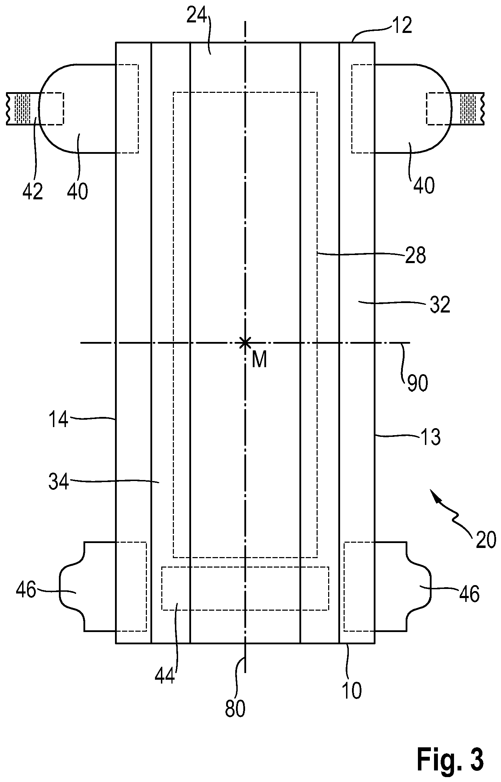

The absorbent article comprises a liquid permeable topsheet 24 on its wearer-facing surface, a liquid impermeable backsheet 25 on its garment-facing surface and an absorbent core 28 between the topsheet and the backsheet (shown in dotted line in Figs. 2 and 3). The topsheet typically forms the majority of the wearer-contacting surface of the article and is the first layer that the body exudates contact. The topsheet is liquid permeable, permitting liquids to readily penetrate through its thickness. Any known topsheet may be used in the present invention. The backsheet typically comprises a fluid impermeable plastic film, which may be printed with a backsheet pattern, and a low basis weight nonwoven outer cover glued to this impermeable film to give a nicer feel and appearance to the backsheet.

The absorbent article may also typically comprise a fluid acquisition layer and/ or a fluid distribution layer between the topsheet and the absorbent core, which is not represented for simplicity but are present in most diapers, as well as outer barrier cuffs 32 and inner barrier cuffs 34, as is known in the art. The absorbent article may also comprise other usual components if it is desired to increase the performance of the article, such as transverse barrier cuffs, front and/or back elastic waistbands, a lotion application on the topsheet, longitudinally extending channels in the core and/or the distribution layer, a wetness indicator, etc... all these components have been extensively described and exemplified in the art. More detailed disclosures of example of such components are for example disclosed in WO201493323, WO2015/183669 (both Bianchi et al), WO 2015/031225 (Roe et al.) or WO2016/133712 (Ehmsperger et al.) to name a few.

The absorbent article typically comprises a front edge 10, a back edge 12, and two longitudinally-extending side (lateral) edges 13, 14. The front edge 10 is the edge of the article which is intended to be placed towards the front of the user when worn, and the back edge 12 is the opposite edge, and together form the waist opening of the diaper. The lateral edges 13, 14 respectively form the two leg openings. The topsheet 24, the backsheet 25, the absorbent core 28 and the other article components may be assembled in a variety of well-known configurations, in particular by gluing, fusion and/or pressure bonding. The absorbent articles of the invention may comprise any typical layers and components used in absorbent products of the diaper type, and which are not necessarily represented in the simplified Figs. 1-3. A plurality of absorbent articles may be packaged together in a package.

General description of an absorbent core

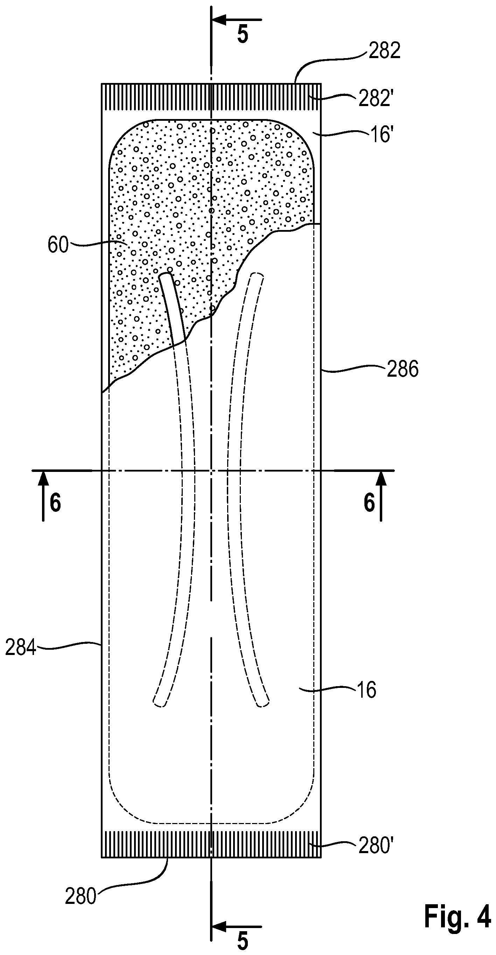

The absorbent core 28 is the component of the absorbent article having the most absorbent capacity. An exemplary absorbent core 28 is shown in isolation in Figs. 4-6, in dry state (before use). The absorbent core may typically have a generally rectangular shape as defined by the longitudinal edges 284, 286 and transversal front edge 280 and back edge 282. The absorbent core 28 comprises an absorbent material 60, deposited as a layer having a generally rectangular outline, as represented on Fig. 4. This absorbent core represented is of course not limiting the scope of the invention as the invention is applicable to a wide variety of absorbent cores. It is also common to have an absorbent material 60 layer having a non-rectangular outline ("shaped" core), in particular the absorbent material layer may define a tapering along its width towards the central region of the core (or "dogbone" shape). In this way, the absorbent material deposition area may have a relatively narrow width in an area of the core intended to be placed in the crotch region of the absorbent article. This may provide for example better wearing comfort. Other shapes can also be used such as a "T" or "Y" or "sand-hour" for the area of the absorbent material.

The absorbent material 60 may be any conventional absorbent material known in the art. For example, the absorbent material may comprise a blend of cellulose fibers and superabsorbent particles ("SAP"), typically with the percentage of SAP ranging from 40% to 70% by weight of the absorbent material. The absorbent material may also be free of cellulose fibers, as is known in so-called airfelt-free cores where the absorbent material consists of SAP.

Various absorbent core designs comprising high amount of SAP have been proposed in the past, see for example in US5,599,335 (Goldman), EP1,447,066 (Busam), WO95/11652 (Tanzer), US2008/0312622A1 (Hundorf), WO2012/052172 (Van Malderen). In particular the SAP printing technology as disclosed in US2006/024433 (Blessing), US2008/0312617 and US2010/0051166A1 (both to Hundorf et al.) may be used. The invention is however not limited to a particular type of absorbent core. The absorbent core may also comprise one or more glue such as auxiliary glue applied between the internal surface of one (or both) of the core wrap layers and the absorbent material to reduce leakage of SAP outside the core wrap. A micro-fibrous adhesive net may also be used in airfelt free cores as described in the above Hundorf references. These glues are not represented in the Figures for simplicity.

The absorbent material may be deposited as a continuous layer within the core wrap. The absorbent material may also be present discontinuously for example as individual pockets or stripes of absorbent material enclosed within the core wrap and separated from each other by material-free junction areas. A continuous layer of absorbent material, in particular of SAP, may also be obtained by combining two absorbent layers having matching discontinuous absorbent material application pattern wherein the resulting layer is substantially continuously distributed across the absorbent particulate polymer material area. As for example taught in US2008/0312622A1 (Hundorf), each absorbent material layer may thus comprise a pattern having absorbent material land areas and absorbent material-free junction areas, wherein the absorbent material land areas of the first layer correspond substantially to the absorbent material-free junction areas of the second layer and vice versa.

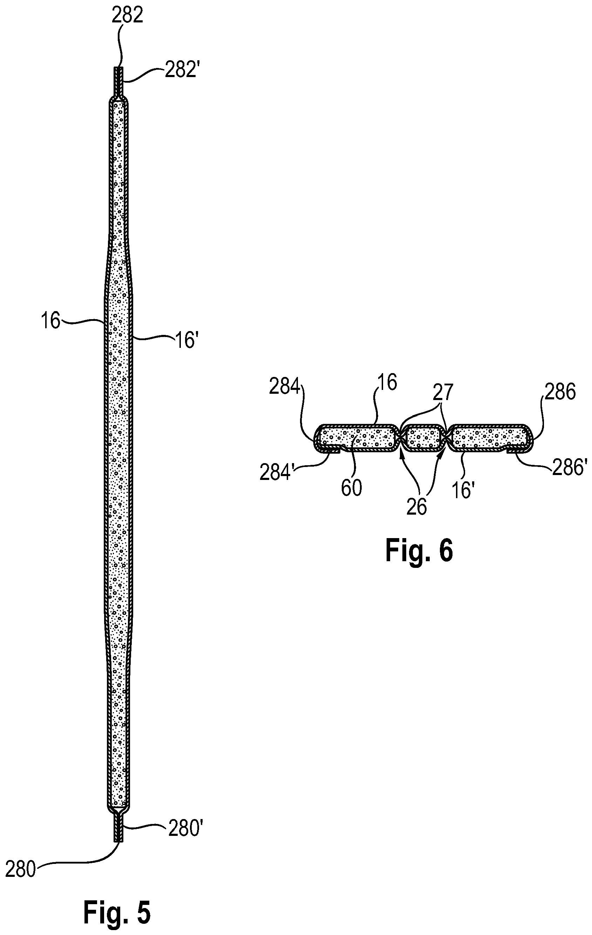

The basis weight (amount deposited per unit of surface) of the absorbent material may also be varied to create a profiled distribution of absorbent material, in particular in the longitudinal direction (as schematically illustrated in Fig. 5) to provide more absorbency towards the center and the middle of the core, but also in the transversal direction, or both directions of the core. The absorbent core may also comprise longitudinally extending channels which are substantially free of absorbent material within the absorbent material area. The core wrap may be bonded through these material-free areas. Exemplary disclosures of such channels in an airfelt-free core can be found in WO2012/170778 (Rosati et al.) and US2012/0312491 (Jackels). Channels may of course also be formed in absorbent cores comprising cellulose fibers.

Core wrap

The function of the core wrap is to enclose the absorbent material. As indicated in the background, different core wrap constructions can be used. Typical core wraps comprise two nonwoven substrates 16, 16', which are attached to another and form respectively the top layer 16 and the bottom layer of the core wrap 16'. These two layers may be typically attached to another along at least part of the periphery of the absorbent core to form a seal. Typically, neither the first nor the second substrate needs to be shaped, so that they can be rectangularly cut for ease of production, but other shapes are not excluded. The terms "seal" and "enclosing" are to be understood in a broad sense. The seal does not need to be continuous along the whole periphery of the core wrap but may be discontinuous along part or the whole of it, such as formed by a series of seal points spaced on a line. Typically, a seal may be formed by gluing and/or thermal bonding.

The core wrap represented in the Figures comprises a top layer 16 which is wider than the bottom layer 16' so that two flaps of the top layer can be folded over the bottom layer along the longitudinal edges 284, 286 of the core respectively to which they are attached, typically by an adhesive to form the longitudinal seals 284', 286'. The front edge 280 and back edge 282 may also be sealed, for example by a sandwich seal 280', 282'. Such transversal seals may for example made by adhesive stripes applied in machine direction by the slot glue technique, as is known in the art. Alternatively, is it possible to leave the transversal edges 280, 282 open without a seal. For example, there may be enough core wrap material between the edges of the core and the absorbent material 60 to provide a buffer zone at these ends.

The invention is applicable to any of these seals, between the core wrap layers, as well as the core channel bonds 27 that will be discussed further below, as these seals or bonds are typically subject to a peel force once the absorbent material swells for a prolonged length of time. Alternatively, the core wrap may be made of a single piece of nonwoven which has been folded over itself around the absorbent material layer 60, and is bonded to itself along a single longitudinal seal, instead of two longitudinal seals 284' and 286' as represented in the Figures. The invention is also applicable to such a core wrap.

The top layer and the bottom layer may be made from the same base substrate material which has been differently treated. Such nonwoven substrate may have a basis weight within a range of from 8 to 12 gsm. The top layer may be typically a nonwoven layer made of synthetic fibers that has been treated with a surfactant to increase its hydrophilicity. Both layers may in particular each comprises or consists of a nonwoven web, such as a carded nonwoven, a spunbond nonwoven ("S") or a meltblown nonwoven ("M"), and a multi-layer of any of these. For example, spunbond / meltblown laminate (spunmelt) polypropylene nonwovens are commonly used and are particularly suitable, especially those having a multi-layer SMS, or SMMS, or SSMMS, structure. Examples are disclosed in US7,744,576, US2011/0268932A1, US2011/0319848A1 or US2011/0250413A1. Typical material used to make the synthetic fibers are PE (polyethylene), PET (polyethylene terephthalate) and in particular PP (polypropylene).

Spunbond, also called spunlaid, nonwovens are made in one continuous process. Fibers are spun through a number of small orifices in a spinneret to form fibers or filaments, which are then directly dispersed into a web by deflectors or can be directed with air streams on a moving foraminous surface, such as a wire mesh conveyor. Meltblown nonwovens are produced by extruding melted polymer fibers through a spinneret or die consisting of up to 40 holes per inch to form long thin fibers which are stretched and cooled by passing hot air over the fibers as they fall from the die. The diameters of the fiber are significantly reduced by hot air which also breaks the continuous filaments into microfibers of varying length to diameter ratio. The extremely fine fibers (typically polypropylene) differ from other extrusions, particularly spunbond, in that they have low intrinsic strength but much smaller size offering key properties.

The spunbond process can be combined with the meltblown process to form a multi-layer web having S (spunbond) layer and M (meltblown) layer, in particular SM, SMS or SMMS webs, which are strong and offer the intrinsic benefits of fine fibers. The nonwovens may be consolidated using known techniques, typically thermal point bonding. In thermal point bonding, heat is applied locally on individual regions of the nonwoven to locally melt and fuse the fibers together. Fusion bond patterns are for example disclosed in US 2011/0250413 (Hu et al.) and US2014/0072767A1 (Klaska et al.). The resultant web is typically collected into rolls at the supplier and subsequently converted to finished products.

Core Channels

The absorbent core 28 may comprise one or more channels, in particular at least two channels 26, within the absorbent material layer. The channels may in particular be areas substantially free of absorbent material, in particular areas completely free of absorbent material (accidental minute amount of absorbent material due to involuntary contamination of the channels due to the high speed of the making process being disregarded).

The channels 26 may comprise a bond 27 between the top side 16 of the core wrap and the bottom side 16' of the core wrap. This bond 27 provides for structural integrity of the channels in dry and wet state. Any known bonding techniques known in the art may be used to provide for this bond, in particular one selected from adhesive bonding, thermo bonding, mechanical bonding, ultrasonic bonding, or any combinations thereof. An adhesive may be for example applied in the areas of the channels on the inner side of the top side and/or the inner side of the bottom side of the core wrap, typically by slot glue application or any other means, followed by the application of pressure in the areas of the channels to provide a good adhesive bonding in these areas. Exemplary patent disclosures of such adhesive bonding processes can be found for an airfelt or airfelt-free absorbent cores in WO2012/170798A1 (Jackels et al.), EP2,905,000 (Jackels et al.) and EP2,905,001 (Armstrong-Ostle et al.).

The present invention may be particularly useful to make these channel bonds 27, in addition or alternatively to the core perimeter bonds 280'-286'. Typically, the bonds 27 may generally have the same outline and shape as the channel areas 26 in which they are contained, but may be slightly smaller to allow for a safety margin (e.g. by a few mm) as some deviations from the optimal registration may happen during high speed process. It is expected that channel bonds 27 may be more efficiently made and stronger if they are provided in macroscopic areas with no absorbent material (except of course accidental contamination) compared to bonds provided in areas containing non-negligible absorbent material.

Pant diaper

The absorbent article may also be in the form of a pant having permanent or refastenable side seams, which is not represented herein but for which the invention may also apply. Pant article comprising refastenable seams are for example disclosed in US2014/0005020 and US9,421,137. Typical pant articles comprise a chassis (sometimes referred to as a central chassis or central panel) comprising a topsheet, a backsheet, and an absorbent core, which may be as disclosed herein, and a front belt that defines a front waist region and a back belt that defines a back waist region. The chassis may be joined to a wearer-facing surface of the front and back belts or to a garment-facing surface of the belts. Side edges of the front belt may be joined to side edges of the back belt to form two side seams. The side seams may be any suitable seams known to those of skill in the art, such as butt seams or overlap seams, for example. When the side seams are permanently formed or refastenably closed, the absorbent article in the form of a pant has two leg openings and a waist opening circumference. The side seams may be permanently joined using adhesives or bonds, for example, or may be refastenably closed using hook and loop fasteners, for example.

Alternatively, instead of attaching belts to the chassis to form a pant, discrete side panels may be attached to side edges of the chassis. Suitable forms of pants comprising discrete side panels are e.g. disclosed e.g. in US6,645,190; US8,747,379; US8,372,052; US8,361,048; US6,761,711; US6,817,994; US8,007,485; US7,862,550; US6,969,377; US7,497,851; US6,849,067; US6,893,426; US6,953,452; US6,840,928; US8,579,876; US7,682,349; US7,156,833; and US 7,201,744.

Backsheet

The backsheet 25 is the liquid impermeable layer that generally form the garment-facing side of the absorbent article. The backsheet 25 prevents, or at least inhibits, the bodily exudates absorbed and contained in the absorbent core 10 from soiling articles such as bedsheets, undergarments, and/or clothing. The backsheet typically comprises a liquid impermeable, or at least substantially liquid impermeable layer, such as a thin plastic film having a thickness of about 0.012 mm to about 0.051 mm. Suitable backsheet materials also include breathable materials which permit vapors to escape from the absorbent article, while still preventing, or at least inhibiting, bodily exudates from passing through the backsheet.

The backsheet 25 typically further comprises on its external side a nonwoven outer cover for improving the overall feel of the backsheet. The outer cover material (sometimes referred to as a backsheet nonwoven) is typically a nonwoven material joined to and covering the backsheet film. Thus the outer cover material typically forms at least a portion of the garment-facing surface of the absorbent article 20. The outer cover material may comprise a bond pattern, apertures, and/or three-dimensional features.

Landing Zone

Referring to Figs. 1 and 2, the absorbent article 20 in the form of a taped diaper may have a discrete landing zone 44 on its garment-facing side, typically disposed proximate the front edge 10 of the article 20. The landing zone 44 is configured to receive the fasteners 42 and may comprise, for example, a plurality of loops configured to be engaged with, a plurality of hooks on the fasteners 46, or vice versa.

The landing zone 44 typically comprises one or more discrete nonwoven materials that are attached to a portion of the outer cover material 40 in the front waist region 12. The present invention is in particular applicable to the bond area between such a landing zone 44 and the backsheet outer cover 25.

Bond areas

The absorbent article 20 comprises a bond area between a first nonwoven material and a second nonwoven material. The bond area may be continuous or discontinuous. A polymeric filler composition according to the invention is disposed within the bond area. The polymeric filler composition may be the only bonding means holding the first nonwoven material and the second nonwoven material bonded together within the bond area. Alternatively, the polymeric filler composition may be supplemented by another bonding means, such as mechanical bonds or fusion bonds. However, it is preferred that the bonding area is free of conventional hotmelt adhesive, which comprises a substantial amount of tackifier, e.g. more than 10% by weight of tackifiers.

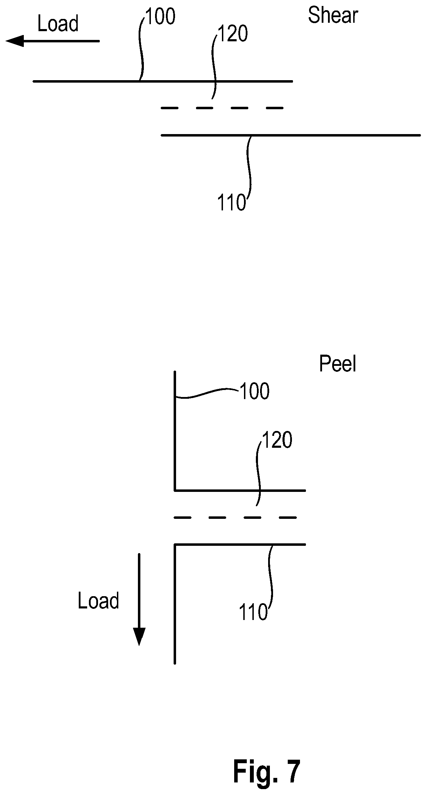

While the following list is not limiting, it was found that the polymeric filler composition of the invention was particularly efficient to form bond between nonwovens subjected to a creep force or load during usage of the absorbent article. Schematically, shear forces are applied to at least one of the layers 100, 110 in a plane parallel to the bond area, while peel forces are applied to a first layer 100 and/or a second layer 110 in a plane perpendicular to the bond area 120 (see illustration in Fig. 7).

The present invention is applicable to any pair of nonwovens of the articles where at least some peeling constraint is applied for at least some time during the normal usage of the article. In particular, the first nonwoven material and the second nonwoven material can be a pair selected from the top layer 16 and bottom layer 16' of the core wrap, or the landing zone 40 and the outer cover of the backsheet 25.

Other nonwoven materials that may be used in the present invention may be selected in the group consisting of the liquid permeable topsheet 24, the barrier leg cuff material 32-34, any nonwoven waist bands (not shown), an acquisition material or secondary top sheet, or any other nonwoven materials.

The polymeric filler composition may be typically present at a basis weight ranging from about 13 gsm to about 30 gsm within the bond area, alternatively from about 15 gsm to about 25 gsm. The first and/or the second nonwoven material may comprise natural or recycled fibers.

It may be that the adhesive is applied in a way that the bond area is not uniformly covered with the adhesive but there can e.g. be parts of the bond area which are free of adhesive, e.g. if the adhesive has been applied in stripes by using a slot coating nozzle with a comb shim. In such cases, the basis weight of the adhesive is defined as the average basis weight of the adhesive over the bond area.

Polymeric filler composition

The polymer filler composition of the invention comprises at least one polymer selected from polypropylene homopolymers, propylene-ethylene copolymers, and mixtures thereof. The polymer is at least the main component of the polymeric filler composition, which may comprise at least 50% of the polymer by weight of the polymer filler composition. The polymeric filler composition may in particular comprises at least 60%, or at least 70%, or at least 80% or at least 90% and up to 100%, or up to 99.5%, or up to 99%, of the polymer, by weight of the composition. For simplification of the compounding process, it may be advantageous that the polymer filler composition consists essentially of an unblended polymer.

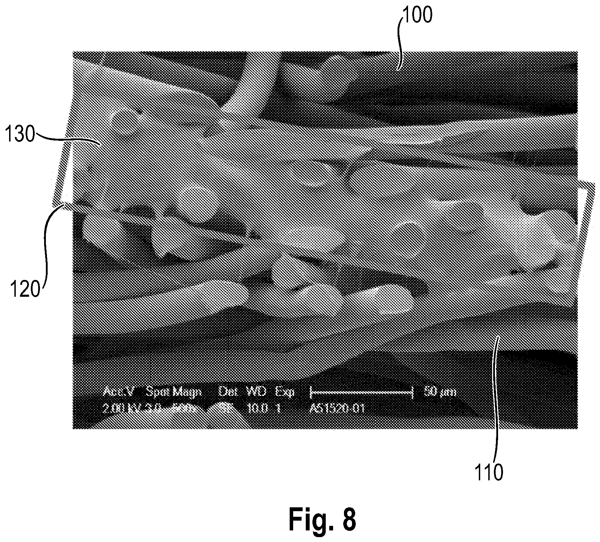

While not wishing to be bound by theory, the inventors believe that in order to provide for strong bonds between a first nonwoven 100 and a second nonwoven 110, the polymeric filler composition 130 should enable the right microscopic pattern in the bond area 120, which can be described as a dual row entanglement of adjacent fibers from both nonwovens (as illustrated in the electron microscopic of Fig. 8). Ideally, the majority of the fibers is embedded by 360°, or at least by 180°. This creates a mechanical lock in which the fibers are cemented. The inventors found that the combination of such mechanical lock provided by such structure in combination with the high Toughness of the polymer enables a bond which is resistant against peel creep under in-use conditions. Therefore, also pure polymers like Licocene 2502, which are non-tacky at room temperature were found suitable for the present invention at least when they are applied to the two nonwovens in the microscopic structure described before.

While the polymers indicated above (polypropylene homopolymers, or propylene-ethylene copolymers) can be generally used to form such inter-fibrous locking, the present inventors have found that not all such polymers provide the desired creep resistance properties between two nonwoven substrates. After testing and comparison of various polymeric filler compositions, the inventors have found that the polymeric filler composition should further have certain mechanical properties designated as Toughness and Yield Stress at usage temperature (from 23°C to 37°C), which will be illustrated below in the form of different examples.

Crystallinity of the propylene-ethylene copolymers is believed to be a contributor to the Toughness. The propylene-ethylene copolymer is advantageously semi-crystalline, so that the filler composition has an enthalpy of fusion of at least 20 J/g, as measured according to the Enthalpy of Fusion Measurement Method described herein. However too high crystallinity can make the polymer brittle, so the enthalpy of fusion may be advantageously less than 100 J/g, in particular less than 50 J/g, as measured according to the Enthalpy of Fusion Measurement Method described herein.

Commercial example of suitable propylene-ethylene copolymers is Clariant's Licocene® PP 2502, which has a measured enthalpy of fusion of 29.4 J/g. On the other hand, Licocene® PP 1502 which has a measured enthalpy of fusion of 15.1 J/g or Licocene® PP 1602 which has a measured enthalpy of fusion of 16.7 J/g, are believed to be not crystalline enough.

The enthalpy of fusion is however believed not to be the only relevant factor to predict good peel creep resistance of the polymers. For example, Idemitsu's L-MODU S-410 has a measured enthalpy of fusion of only about 2 J/g but still has a good performance. The inventors believe that the polymer filler composition's performance is driven by a relatively high Toughness, as measured as indicated below. This Toughness is believed to be driven by the homopolymer nature of L-MODU S410 as well as the relatively higher molecular weight of the polymer (45.000 g/mol). Toughness is believed to be increased with the molecular weight.

The polymer comprises propene monomer units. In copolymers, the percentage of propene monomer units may range for example from 50% to 99% by weight of the copolymer. If the percentage of propene monomer units is not known, it may be determined by a suitable method, such as nuclear magnetic resonance or infrared spectroscopies, as known to those of skill in the art.

The polymers of the invention can be prepared using a metallocene catalysts. The polymer can be for example prepared by the methods described in US2016/0053149A1 ("*Ready-to-use hot melt adhesive having an improved property profile*"). The polymer can be prepared into a final polymeric filler composition by heating the primary polymer to elevated temperatures (e.g., about 135 to about 175° C) that melts the polymer. Once molten, one or more optional ingredients (e.g., additive or other polymers components) can be added to the primary polymer. A mixer can be used to mix the components together into a final polymeric filler composition. See for example US5,723,546, which discloses such blending.

According to the invention, the polymeric filler composition comprises less than 5 %, alternatively less than 3 %, alternatively less than 2 %, alternatively less than 1 %, alternatively less than 0.5 %, and alternatively less than 0.1 % of a tackifier, by weight of the polymer filler composition. Exemplary tackifiers can include aliphatic hydrocarbon resins, aromatic modified aliphatic hydrocarbon resins, hydrogenated poly-cyclopentadiene resins, poly-cyclopentadiene resins, gum rosins, gum rosin esters, wood rosins, wood rosin esters, tall oil rosins, tall oil rosin esters, poly-terpenes, aromatic modified poly-terpenes, terpene-phenolics, aromatic modified hydrogenated poly-cyclopentadiene resins, hydrogenated aliphatic resins, hydrogenated aliphatic aromatic resins, hydrogenated terpenes and modified terpenes, and hydrogenated rosin esters. The polymeric filler composition can be free of a tackifier.

There are significant advantages to minimizing or avoiding the use of a tackifier as it may reduce the cost of the polymeric filler composition, as well as eliminate an additional ingredient and potential issues that may be associated with supplying the additional ingredient. Furthermore, tackifiers may impart undesirable odor in disposable articles and can also act as carriers of low molecular weight plasticizers (e.g., process oils that are used in SBC based adhesives) that may weaken the polyethylene back sheet materials used in absorbent articles and textile articles.

The polymeric filler composition may optionally comprise additives such as an antioxidant, UV stabilizer, brightener, colorant, fragrance etc... The polymeric filler composition may comprise less than 5% by weight of such additives. Any antioxidant known to a person of ordinary skill in the art may be used in the adhesion composition.

Non-limiting examples of suitable antioxidants include amine-based antioxidants such as alkyl diphenyl amines, phenyl-naphthylamine, alkyl or aralkyl substituted phenyl-naphthylamine, alkylated p-phenylene diamines, tetramethyl-diaminodiphenylamine and the like; and hindered phenol compounds such as 2,6-di-t-butyl-4-methylphenol; 1,3,5-trimethyl-2,4,6-tris(3',5'-di-t-butyl-4'-hydroxybenzyl)benzene; tetra kis [(methylene(3,5-di-t-butyl-4-hydroxyhydrocinnamate)]methane (e.g., IRGANOXTM 1010, from Ciba Geigy, New York); octadecyl-3,5-di-t-butyl-4-hydroxycinnamate (e.g., IRGANOXTM 1076, commercially available from Ciba Geigy) and combinations thereof. The amount of the antioxidant in the polymeric filler composition may be equal to or less than 1 %, alternatively from about 0.05 % to about 0.75 %, and alternatively from about 0.1 % to about 0.5 %, by weight of the polymeric filler composition.

The polymeric filler composition may optionally comprise a UV stabilizer, that may prevent or reduce the degradation of the composition by radiation. Any UV stabilizer known to a person of ordinary skill in the art may be used in the polymeric filler composition. Non-limiting examples of suitable UV stabilizers include benzophenones, benzotriazoles, aryl esters, oxanilides, acrylic esters, formamidine carbon black, hindered amines, nickel quenchers, hindered amines, phenolic antioxidants, metallic salts, zinc compounds, and combinations thereof. Where used, the amount of the stabilizer in the polymeric filler composition can be less than 1 %, alternatively from about 0.05 % to about 0.75 %, and alternatively from about 0.1 % to about 0.5 %, by weight of the polymeric filler composition

The polymeric filler composition may optionally comprise a brightener, colorant, and/or pigment. Any colorant or pigment known to a person of ordinary skill in the art may be used in the polymeric filler composition. Non-limiting examples of suitable brighteners, colorants, and/or pigments include fluorescent materials and pigments such as triazine-stilbene, coumarin, imidazole, diazole, titanium dioxide and carbon black, phthalocyanine pigments, and other organic pigments such as IRGAZINB, CROMOPHTALB, MONASTRALB, CINQUASIAB, IRGALITEB, ORASOLB, all of which are available from Ciba Specialty Chemicals, Tarrytown, N.Y. Where used, the amount of the brightener, colorant, and/or pigment in the polymeric filler composition can be less than 10 %, alternatively from about 0.01 % to about 5 %, and alternatively from about 0.1 % to about 2 %, by weight of the polymeric filler composition.

The polymeric filler composition may optionally comprise a fragrance such as a perfume or other odorant. Such fragrances may be retained by a liner or contained in release agents such as microcapsules that may, for example, release fragrance upon removal of a release liner from or compression on the adhesive composition. Where used, the amount of the fragrance in the polymeric filler composition can be less than 3 %, alternatively less than 2 %, alternatively less than 1 %, alternatively from about 0.05 % to about 0.75 %, and alternatively from about 0.1 % to about 0.5 %, by weight of the polymeric filler composition.

EXAMPLES & DATA

Exemplary nonwoven-nonwoven laminates were made as follows.

A first nonwoven material and a second nonwoven material are bonded via a specified slot coating process using a bonding material to form a laminate. The bonding material may be a polymeric filler composition as claimed or a comparative example such as a tackifier-containing adhesive composition. The first nonwoven material and the second nonwoven material used to form the laminate are 15 gsm polypropylene spunbond (SSS) with a thermal bond pattern matching that of US Design Patent D714,560 that covers approximately 13-15% of first nonwoven material and the second nonwoven material. The first nonwoven material and the second nonwoven material are provided in roll stock form and are 230 mm in width.

The bonding material is slot coated onto the first nonwoven material at a speed of 114 m/min and the total tension at the point of application is 0.5 lbs (10.5 N/m tension per unit width). The bonding material is slot coated onto the first nonwoven material using an ITW Dynatec APEX Slot Die, from ITW Dynatec, Hendersonville TN, USA, or equivalent). The shim of the die is 0.15 mm thick and cut such that a coating of bonding material is applied in the cross-machine direction of 27mm width and continuous in the machine direction. The bonding material flow rate for the nozzle is set such that the applied basis weight can be chosen, in the present examples at $10 \pm 0.1$ gsm.

The bonding material is maintained at a temperature $\pm 5$ °C at all points up to and including the applicator such that the viscosity of the of bonding material at the maintained temperature is within the range of 1,500 mPa·s to 10,000 mPa. s.

The overall slot coating process is performed at an ambient temperature of $21 \pm 2$ °C. The bonding material is applied to the first nonwoven material with the slot coat die by bringing the slot coat die into contact with the first nonwoven material supported between two non-driven web-support idlers that co-rotate with the moving first nonwoven material. Each web-support idler has a 50 mm diameter. The spacing of the web-support idlers is set to 200 mm, center to center, and the bonding material applicator's exit is set at a point 150 mm from the downstream idler's center. The bonding material applicator is pressed into the tensioned first nonwoven material between the idlers, such that the first nonwoven material is deflected 3-4 mm at the exit point of the bonding material from the slot coat die, with respect to the plane made by the first nonwoven material under tension when the bonding material applicator is absent. The angle made between the slot coat die's shim plane and the plane of the tensioned first nonwoven material when the bonding material applicator is not engaged is the pitch angle. This angle is described to be zero pitch when the planes are perpendicular to each other. During application of the bonding material to the first nonwoven material, the angle is set to zero pitch. In other words, the plane of the shim relative to the plane of the tensioned first nonwoven material when the bonding material applicator is not engaged is 90° on the side of the downstream web-support idler. The bonding material coating is centered along the length of the first nonwoven material by centering the width of the slot coat die on the cross-machine width of the first nonwoven material.

The coated first nonwoven material is then brought into contact with the second nonwoven material 165 mm after the point of coating with the bonding material to create a laminate. A compression nip is used to compress the laminate at a point 955 mm from the point of coating with the bonding material. The compression nip consists of a steel roll and a rubber coated steel roll that coaxially counter rotate while in contact with each other to create pressure between them. The steel roll and rubber roll diameters are 100 mm and the rubber coating is 20 mm thick with a Shore A hardness of 100. The steel roll and rubber coated steel roll are forced together to develop a constant pressure of 70 psi in the nip. The laminate travels through the nip and is subjected to this pressure as it travels. The laminate speed and the resulting bonded laminate is maintained at 114 m/min. After passing through the compression nip, the bonded laminate is wound onto a roll for sampling at 1.5 lbs winding tension. Laminated test panels are immediately cut from the wound roll of bonded laminate. The laminated test panels are equilibrated at $21 \pm 2$ °C and 40% relative humidity for a minimum of 24 hours before further preparation and testing.

Performance

Laminates obtained as described above using commercial compositions were tested according to Static Peel Force Time test described below, in which a constant peel force is exerted by a weight of ca. 200g on one layer of the laminate. Toughness and Yield Stress were also measured for each composition according to Extensional Test Method described below.

Table 1 below shows the values measured for various tackifier-free compositions. Licocene designates a range of metallocene-technology based propylene-ethylene copolymer. L-MODU designates a range of metallocene catalyst homopolymer polypropylene from Idemitsu. RT designates a range of Amorphous Poly Alpha Olefin (APAO) from Rextac.

Table 1

| Polymer | Source | Performance [min / 10 mm bonded length] | Toughness [MJm$^{-3}$] | Yield Stress [MPa] |
|---|---|---|---|---|
| Licocene 2502 | Clariant | >740.7 * | 28.0 | 9.0 |
| L-MODU 5410 | Idemitsu | >740.7 * | 84.7 | 7.5 |
| Licocene 1602 | Clariant | Not measured | 26.1 | 4.7 |
| RT2830 | Rextac | 3.1 | 22.9 | 2.9 |
| Licocene 1302 | Clariant | n.a. ** | 2.9 | 4.8 |
| Licocene 1502 | Clariant | n.a. ** | 9.1 | 2.5 |
| * testing stopped as no failure observed; ** not measured, these materials are very soft with low crystallinity (low enthalpy of fusion) and are expected to be not suitable a peel resistant filler composition; | | | | |

The Static Peel Force Time test was also conducted with commercially available tackifier-containing hotmelt adhesives, as shown in Table 2 below.

Table 2

| Polymer | Source | Performance [min / 10 mm bonded length] | Toughness [MJm$^{-3}$] | Yield Stress [MPa] |
|---|---|---|---|---|
| DM3800 | Henkel | 7.6 | 40.5 | 0.8 |
| 2401 | Bostik | 135.6 | n.a. | n.a. |

The inventors believe that the polymer filler composition should have a Toughness of at least 25 MJm$^{-3}$ and preferably a Yield Stress of at least 5 MPa in order to provide the bond with a long Static Peel Force Time, especially a lower basis weight (10-20 gsm). Toughness and Yield Stress parameters, characterize the behavior of the bonding composition submitted to a high strain, as opposed to previous methods describing adhesives using oscillatory rheology (e.g. the storage modulus G'). The inventors have found that these large are critical to predict the adhesive behavior according to the static test method (hang time), which again is needed to predict the performance of the bonds in use in the diaper.

The Toughness of the polymer filler composition may be at least 27 MJm$^{-3}$. The Toughness may also be up to 200 MJm$^{-3}$, or up to 150 MJm$^{-3}$ or up to 100 MJm$^{-3}$ or up to 60 MJm$^{-3}$.

TEST METHODS

Extensional Test Method

The Extensional Test Method is used to determine the Yield Stress and the Toughness for a specimen of a polymer composition. A thin film specimen formed of polymer composition is analyzed with a rotational rheometer fitted with a specialized fixture with counter rotating rollers, and the stress associated with extensional strain imparted is measured and recorded.

Instrumental setup

A rotational rheometer (ARES G2, TA Instruments, New Castle, DE, USA, or equivalent) is fitted with a fixture that has counter rotating cylindrical rollers specifically designed for the interrogation of extension deformation of films. An example of a suitable fixture is the Extensional Viscosity Fixture, or EVF (EVF, TA Instruments, or equivalent). The rheometer is further fitted with a forced-convection oven FCO (FCO, TA Instruments, or equivalent) and cooling system (ACS 2, TA Instruments, or equivalent) capable of controlling temperate from at least -50 to 250 °C to a within a tolerance of 0.5 °C.

Specimen preparation

Approximately 6 g $\pm$ 2 g of the polymer composition is placed in a circular polytetrafluoroethane (PTFE) bowl with a flat bottom (diameter of 60 mm $\pm$ 2 mm) and introduced into a vacuum oven held at 170°C. After 15 minutes at ambient pressure, the pressure is lowered to 10 mbar, and the polymer composition is subsequently held at 170°C and at 10 mbar for 45 minutes to remove air bubbles from the polymer composition. If 170°C is insufficient to melt the polymer compositions a temperature 30 $\pm$ 10 °C above the melting temperature of the polymer material composition is used. The polymer composition is removed from the vacuum oven and allowed to cool to ambient lab conditions (23 $\pm$ 2 °C) for 90 $\pm$ 30 minutes, at which point the polymer composition is removed from the PTFE bowl and placed between 2 sheets of siliconised paper (such as product number 114918, Mondi Group, Hilm, Austria, or equivalent). A metal shim 500 $\pm$ 30 $\mu$m in thickness is used in the heated press as a spacer to obtain a film thickness of 500 $\mu$m when pressed with a heated press at 90 °C for 60 seconds at a pressure sufficient to form a polymeric film. If 90 °C is insufficient to press a uniform flat film, a temperature approximately 10 $\pm$ 5 °C below the melting point of the sample material composition such that the sample material composition is in a semi-solid state is used. The film is stored at least 120 hours in the laboratory at 23 $\pm$ 2 °C prior to testing. From the film individual specimens for measurement are punched with a sample cutter to the final specimen dimensions of 20.0 mm by 10.0 mm by 500 $\mu$m.

Measurement

To secure the film to the cylinders of the EVF, the cylinders are heated to 50 °C for 90 $\pm$ 30 s in the forced-convection oven of the rheometer. After opening the oven, a specimen of polymer composition is briefly pressed onto the cylinders of the EVF to secure it to the cylinder surface. The specimen is placed perpendicular to the axis of rotation of the cylinders.

The specimen mounted on the EVF is then placed in the forced convection oven of the rheometer for thermal conditioning and is kept isothermal at 23 $\pm$ 1 °C for 300 $\pm$ 10 s. After this time has elapsed, the specimen is mechanically conditioned. To mechanically condition the specimen, the torque transducer is zeroed, and the sample is put under a pre-stretch rate of 0.001 s$^{-1}$ for 0.30 s and then allowed to relax for 60 s (in this method, all strain is expressed in terms of Hencky strain, also known as "true strain" or "logarithmic strain.").

The measurement is performed in the FCO oven at 23 °C $\pm$ 0.5 °C. The strain rate extension for the measurement is 1 s$^{-1}$, and the strain at maximum extension is 4.0. After measurement, the specimen is checked for rupturing. If it has ruptured, the location of the break is noted. If the rupture is approximately in the middle between the two cylinders of the EVF, the data collected are deemed acceptable. Otherwise, if the polymeric film break is at or close to the rotating cylinders, the results are discarded, and the measurement performed again on a replicate specimen.

Analysis

For the extensional stress calculation, a constant volume is assumed. From the raw torque versus angular displacement data recorded by the rheometer, extensional stress (in megapascals, or MPa) versus Hencky strain data are calculated. The data are plotted in semilogarithmic fashion with Hencky strain on the abscissa (linear scale) and extensional stress on the ordinate (logarithmic scale). A linear range is sought in this plot. If a linear range above a strain of 0.3 can be identified and this range can be fit with a positive slope with an $R^2$ value of 0.98 or greater, the value of the fitted line at a Hencky strain of zero (that is, the y-intercept), is defined as the Yield Stress, which is reported in MPa to the nearest kilopascal. Otherwise, the maximum value of extensional stress recorded during the measurement is reported as the Yield Stress, again reported in MPa to the nearest kilopascal.

The extensional stress (MPa) versus Hencky strain data calculated above are again plotted, but this time in linear fashion with Hencky strain on the abscissa (linear axis) and extensional stress on the ordinate (linear axis). The integral of extensional stress with strain (that is, the area under the extensional stress curve as a function of strain) is calculated from a strain of zero to the strain at which the sample ruptured (or, in the case it did not rupture during the measurement, to a strain of 4.0) and is reported as the Toughness, which is reported in units of megajoules per cubic meter, or MJ m$^{-3}$.

Static Peel Force Time Test Method

The Static Peel Force Time Test Method is used to determine the time required for an adhesive bond to completely delaminate in an approximate 180° peel geometry under constant load and at fixed temperature. Multiple specimens of a representative sample are analyzed for a given sample polymer filler material to establish the Static Peel Force Time.

Sample preparation

If, for a sample polymer filler of interest, an exemplary laminate is available the preparation of which is described above, ten specimens measuring 25.4 mm in the machine direction are taken at random from the equilibrated roll stock. The laminate cross direction (the direction parallel to which the peel is performed in this method) is 27 mm. If, for a sample polymer filler material of interest, an exemplary laminate is not available, two-layer laminate bond specimens of one or more different geometries excised from available materials may be measured. For example, if bond containing filler of interest is present on finished absorbent articles, ten specimens of bonds rectangular in shape may be extracted from ten individual like articles selected at random from a commercially sourced package of diapers. In this case, specimens must contain bonds that measure at least 10 mm parallel to the axis along which the peel will be performed and 25.4 mm transverse to the axis. For each specimen, the dimension of the bonded area parallel to the axis along with the peel is measured to the nearest 1 mm and recorded. Specimens must further have sufficient unbonded material in both laminate layers on at least one side of the bonded area along the axis of peel such that material can be affixed to wooden dowel rods as discussed below.

Regardless of whence specimens for peel analysis are sourced, each of the unbonded layers at the edge of the laminate is separately folded over a small round wooden dowel rod 2 mm in diameter and approximately 40 mm long and the wrapped dowels are secured with a 2 inch wide bulldog clip. The clip is placed over the wrapped dowel and clamped onto the doubled layer of material such that the material does not slip or pull out of the clip.

Measurement

With clips attached, the test specimens are placed in preconditioned incubator (at 38 ± 1 °C) for about 2 hours before testing. After 2 hours, each sample is suspended in the chamber by the clip attached to one laminate layer, and a weight is attached to the other laminate layer's clip, hanging therefrom. The hanging weight, the bulldog clip, and the dowel have a total mass of 200 ± 2 g.

The specimen is suspended such that the bottom of the attached weight is located high enough above the bottom of the chamber so that the entire laminate can peel apart, and the weight can freely fall to the bottom of the chamber through some remaining distance. A timer is used to measure the time between the time at which the hanging weight is attached and the time at which the bonded area of the test laminate fully delaminates. For each specimen, this time to failure is recorded to the nearest second.

Analysis and reporting

For each specimen, the time to failure is normalized to a 10 mm bond dimension to establish that specimen's normalized hang time, recorded for each specimen to the nearest second.

$$\text{Normalized hang time [min]}$$
$$= \frac{10 \text{ mm}}{\text{Bond dimension [mm] parallel to axis of peel}} \times \text{time to failure [min]}$$

The arithmetic mean of the normalized hang time values for the ten specimens is calculated and reported as the Static Peel Force Time in minutes to the nearest minute.

Viscosity Test Method

The Viscosity Parameter of the polymer filler composition is determined using the Viscosity Parameter Test Method, which consists of performing ASTM D3236-15 with the following additional guidance. A Brookfield RVT viscometer with spindle SC 4-27 (Brookfield Engineering, Middleboro, MA, USA), or equivalent, is used. The sample temperature is maintained at 170.0 ± 1.0 °C, unless otherwise specified, throughout the measurement. The sample is preheated for 10 minutes and stirred with the measurement spindle for 30 min. The spindle is rotated at 20 rpm throughout the measurement. The resulting apparent viscosity, as described in section 10, is reported as the "viscosity" in units of millipascal-seconds to the nearest 100 mPa·s.

Enthalpy of Fusion Measurement Method

The Enthalpy of Fusion of a hot-melt adhesive composition is determined using the Enthalpy of Fusion Test Method, which consists of performing ASTM D3418-15 with the following additional guidance. Hot-melt specimen(s) are preferably extracted from molded or pelleted raw material adhesive composition. If raw material is not available, specimen(s) of adhesive are extracted from bonds of interest in an absorbent article using techniques known to those of skill in the art. Dry nitrogen is used as the purge gas in the differential scanning calorimeter (DSC). The rate of increase of temperature in the DSC is 10 °C/min, and the rate of decrease of temperature in the DSC is 1 °C/min. The mass-normalized enthalpy of fusion is calculated as specified in section 11.4 based on the curve corresponding to decreasing temperature (at 1 °C/min) and is reported as the "Enthalpy of Fusion" in units of joules per gram (J/g) to the nearest 0.1 J/g.

**Claims**

1. An absorbent article (10) comprising:

   a) a first nonwoven material (100);
   b) a second nonwoven material (110);
   c) at least one bond area (120) between the first nonwoven material and the second nonwoven material; and
   d) a polymeric filler (130) composition disposed within the bond area, wherein the polymeric filler composition comprises:

   - one or more polymer(s) selected from the group consisting of polypropylene homopolymers, propylene-ethylene copolymers, and mixtures thereof; and
   - from 0 % to less than 5 % of a tackifier, by weight of the polymeric filler composition; and wherein the polymer filler composition has a Toughness of at least 25 $MJ.m^{-3}$, wherein Toughness is measured according to the Extensional Test Method described herein

2. An absorbent article according to claim 1, wherein the polymeric filler composition has a Yield Stress of at least 5 MPa, wherein Yield Stress is measured according to the Extensional Test Method described herein.

3. An absorbent article according to any of the preceding claims, wherein the polymeric filler composition consists of an unblended polymer which is a polypropylene homopolymer or a propylene-ethylene copolymer, and optionally antioxidant stabilizer, perfumes and/or pigments.

4. An absorbent article according to any of the preceding claims, wherein the polymeric filler composition is present at a basis weight of from 5 $g/m^2$ to 30 $g/m^2$ in the bond area, in particular at a basis weight of from 10 $g/m^2$ to 20 $g/m^2$ in the bond area.

5. An absorbent article according to any one of the preceding claims, wherein the first nonwoven is a nonwoven landing zone (44) and the second nonwoven is a backsheet outer cover (25) of the absorbent article.

6. An absorbent article according to any one of claims 1-4, wherein the first nonwoven material is a core wrap top layer (16) and the second nonwoven material is a core wrap bottom layer (16').

7. An absorbent article according to the preceding claim, wherein the bond area is part of the perimeter core bond (280', 282', 284', 286').

8. An absorbent article according to claim 7, wherein the bond area is a core channel bond area (27).

9. An absorbent article according to any of the preceding claims, wherein the viscosity of the polymeric filler composition at 170 °C ranges from about 1,000 mPa·s to about 5,000 mPa·s, as measured according to the Viscosity Test Method as described herein.

10. An absorbent article according to any of the preceding claims, wherein the first and/or the second nonwoven material comprise natural or recycled fibers.

11. An absorbent article according to any of the preceding claims, wherein the bond area has a Static Peel Force Time of least 500 minutes per 10 mm bonded length, as measured according to the Static Peel Force Time Test Method described herein.

12. A package comprising a plurality of absorbent articles according to any of the preceding claims.

13. A first nonwoven material (100) and a second nonwoven material (110) having at least one bond area (120) between the first nonwoven material and the second nonwoven material, wherein a polymeric filler (130) composition is disposed within the bond area, and wherein the polymeric filler composition comprises:

- one or more polymer(s) selected from the group consisting of polypropylene homopolymers, propylene-ethylene copolymers, and mixtures thereof; and
- optionally from 0 % to less than 5 % of a tackifier, by weight of the polymeric filler composition; and

wherein the polymer filler composition has a Toughness of at least 25 MJ.m$^{-3}$, wherein Toughness is measured according to the Extensional Test Method described herein.

14. A process for bonding a first nonwoven material to a second nonwoven material, comprising the step of applying a polymeric filler composition on the first nonwoven material to a bonding area and bonding the second nonwoven material to the first nonwoven material in the bonding area, wherein the polymeric filler composition comprises:

- one or more polymer(s) selected from the group consisting of polypropylene homopolymers, propylene-ethylene copolymers, and mixtures thereof; and
- from 0 % to less than 5 % of a tackifier, by weight of the polymeric filler composition;

wherein the polymer filler composition has a Toughness of at least 25 MJ.m$^{-3}$, wherein Toughness is measured according to the Extensional Test Method described herein.

15. Use of a polymeric filler composition to increase the creep resistance of bond area between a first nonwoven material and a second nonwoven in an absorbent article (100); wherein the polymeric filler (120) composition is disposed within the bond area, and wherein the polymeric filler composition comprises:

- one or more polymer(s) selected from the group consisting of polypropylene homopolymers, propylene-ethylene copolymers, and mixtures thereof; and
- from 0 % to less than 5 % of a tackifier, by weight of the polymeric filler composition;

wherein the polymer filler composition has a Toughness at 23°C of at least 25 MJ.m$^{-3}$, wherein the Toughness is measured according to the Extensional Test Method described herein.

**Patentansprüche**

1. Absorptionsartikel (10), umfassend:

a) ein erstes Vliesmaterial (100);
b) ein zweites Vliesmaterial (110);
c) wenigstens einen Bindungsbereich (120) zwischen dem ersten Vliesmaterial und dem zweiten Vliesmaterial; und
d) eine Polymerfüllmittel(130)-Zusammensetzung, die innerhalb des Bindungsbereichs angeordnet ist, wobei die Polymerfüllmittelzusammensetzung umfasst:

- ein oder mehrere Polymer(e), ausgewählt aus der Gruppe bestehend aus Polypropylenhomopolymeren, Propylenethylencopolymeren und Mischungen davon; und
- zu 0 Gew.-% bis weniger als 5 Gew.-% der Polymerfüllmittelzusammensetzung ein Klebrigmacher; und wobei die Polymerfüllmittelzusammensetzung eine Zähigkeit von wenigstens 25 MJ.m$^{-3}$ aufweist, wobei die Zähigkeit nach dem hierin beschriebenen Dehnprüfverfahren gemessen wird.

2. Absorptionsartikel nach Anspruch 1, wobei die Polymerfüllmittelzusammensetzung eine Fließspannung von wenigstens 5 MPa aufweist, wobei die Fließspannung nach dem hierin beschriebenen Dehnprüfverfahren gemessen wird.

3. Absorptionsartikel nach einem der vorstehenden Ansprüche, wobei die Polymerfüllmittelzusammensetzung aus einem ungemischten Polymer besteht, das ein Polypropylenhomopolymer oder ein Propylenethylencopolymer und wahlweise ein Antioxidationsstabilisierungsmittel, Duftstoffe und/oder Pigmente ist.

4. Absorptionsartikel nach einem der vorstehenden Ansprüche, wobei die Polymerfüllmittelzusammensetzung zu einem Flächengewicht von 5 g/m$^2$ bis 30 g/m$^2$ in dem Bindungsbereich, insbesondere zu einem Flächengewicht von 10 g/m$^2$ bis 20 g/m$^2$ in dem Bindungsbereich gegenwärtig ist.

5. Absorptionsartikel nach einem der vorstehenden Ansprüche, wobei das erste Vlies eine Vliesauftreffzone (44) ist und das zweite Vlies ein Unterschichtaußenmantel (25) des Absorptionsartikels ist.

6. Absorptionsartikel nach einem der Ansprüche 1 bis 4, wobei das erste Vliesmaterial eine Kernumwicklungsoberteilschicht (16) ist und das zweite Vliesmaterial eine Kernumwicklungsunterteilschicht (16') ist.

7. Absorptionsartikel nach dem vorstehenden Anspruch, wobei der Bindungsbereich Teil der Umfangskernbindung (280', 282', 284', 286') ist.

8. Absorptionsartikel nach Anspruch 7, wobei der Bindungsbereich ein Kernkanalbindungsbereich (27) ist.

9. Absorptionsartikel nach einem der vorstehenden Ansprüche, wobei die Viskosität der Polymerfüllmittelzusammensetzung bei 170 °C in dem Bereich von etwa 1.000 mPa s bis etwa 5.000 mPa s liegt, wie gemessen nach dem Viskositätsprüfverfahren wie hierin beschrieben.

10. Absorptionsartikel nach einem der vorstehenden Ansprüche, wobei das erste und/oder das zweite Vliesmaterial natürliche oder recycelte Fasern umfassen.

11. Absorptionsartikel nach einem der vorstehenden Ansprüche, wobei der Bindungsbereich eine Statik-Schälkraft-Zeit von wenigstens 500 Minuten pro 10 mm gebundener Länge aufweist, gemessen nach dem hierin beschriebenen Statik-Schälkraft-Zeit-Prüfverfahren.

12. Verpackung, umfassend eine Vielzahl von Absorptionsartikeln nach einem der vorstehenden Ansprüche.

13. Ein erstes Vliesmaterial (100) und ein zweites Vliesmaterial (110), die wenigstens einen Bindungsbereich (120) zwischen dem ersten Vliesmaterial und dem zweiten Vliesmaterial aufweisen, wobei eine Polymerfüllmittel(130)-Zusammensetzung innerhalb des Bindungsbereichs angeordnet ist, und wobei die Polymerfüllmittelzusammensetzung umfasst:

- ein oder mehrere Polymer(e), ausgewählt aus der Gruppe bestehend aus Polypropylenhomopolymeren, Propylenethylencopolymeren und Mischungen davon; und
- wahlweise zu 0 Gew.-% bis weniger als 5 Gew.-% der Polymerfüllmittelzusammensetzung ein Klebrigmacher; und
wobei die Polymerfüllmittelzusammensetzung eine Zähigkeit von wenigstens 25 MJ.m$^{-3}$ aufweist, wobei die Zähigkeit nach dem hierin beschriebenen Dehnprüfverfahren gemessen wird.

14. Verfahren zum Verbinden eines ersten Vliesmaterials mit einem zweiten Vliesmaterial, umfassend den Schritt des Aufbringens einer Polymerfüllmittelzusammensetzung auf dem ersten Vliesmaterial auf einen Bindungsbereich und Binden des zweiten Vliesmaterials mit dem ersten Vliesmaterial in dem Bindungsbereich, wobei die Polymerfüllmittelzusammensetzung umfasst:

- ein oder mehrere Polymer(e), ausgewählt aus der Gruppe bestehend aus Polypropylenhomopolymeren, Propylenethylencopolymeren und Mischungen davon; und
- zu 0 Gew.-% bis weniger als 5 Gew.-% der Polymerfüllmittelzusammensetzung ein Klebrigmacher;
wobei die Polymerfüllmittelzusammensetzung eine Zähigkeit von wenigstens 25 MJ.m-3 aufweist, wobei die Zähigkeit nach dem hierin beschriebenen Dehnprüfverfahren gemessen wird.

15. Verwenden einer Polymerfüllmittelzusammensetzung, um die Kriechresistenz des Bindungsbereichs zwischen einem ersten Vliesmaterial und einem zweiten Vlies in einem Absorptionsartikel (100) zu erhöhen; wobei die Polymerfüllmittel(120)-Zusammensetzung innerhalb des Bondbereichs angeordnet ist und wobei die Polymerfüllmittelzusammensetzung umfasst:

- ein oder mehrere Polymer(e), ausgewählt aus der Gruppe bestehend aus Polypropylenhomopolymeren, Propylenethylencopolymeren und Mischungen davon; und
- zu 0 Gew.-% bis weniger als 5 Gew.-% der Polymerfüllmittelzusammensetzung ein Klebrigmacher;
wobei die Polymerfüllmittelzusammensetzung eine Zähigkeit bei 23 °C von wenigstens 25 MJ.m-3 aufweist, wobei die Zähigkeit nach dem hierin beschriebenen Dehnprüfverfahren gemessen wird.

**Revendications**

1. Article absorbant (10) comprenant :

   a) un premier matériau non tissé (100) ;
   b) un second matériau non tissé (110) ;
   c) au moins une zone de liaison (120) entre le premier matériau non tissé et le second matériau non tissé ; et
   d) une composition de charge polymère (130) disposée au sein de la zone de liaison, dans lequel la composition de charge polymère comprend :

      - un ou plusieurs polymères choisis dans le groupe constitué d'homopolymères de polypropylène, de copolymères de propylène-éthylène, et de mélanges de ceux-ci ; et
      - de 0 % à moins de 5 % d'un agent collant, en poids de la composition de charge polymère ; et
      dans lequel la composition de charge polymère a une ténacité d'au moins 25 MJ.m$^{-3}$, dans lequel la ténacité est mesurée selon le procédé d'essai par extension décrit ici

2. Article absorbant selon la revendication 1, dans lequel la composition de charge polymère a une limite d'élasticité d'au moins 5 MPa, dans lequel la limite d'élasticité est mesurée selon le procédé d'essai par extension décrit ici.

3. Article absorbant selon l'une quelconque des revendications précédentes, dans lequel la composition de charge polymère est constituée d'un polymère non mélangé qui est un homopolymère de polypropylène ou un copolymère de propylène-éthylène, et éventuellement d'un agent stabilisant antioxydant, des parfums et/ou des pigments.

4. Article absorbant selon l'une quelconque des revendications précédentes, dans lequel la composition de charge polymère est présente à une masse surfacique allant de 5 g/m$^2$ à 30 g/m$^2$ dans la zone de liaison, en particulier à une masse surfacique allant de 10 g/m$^2$ à 20 g/m$^2$ dans la zone de liaison.

5. Article absorbant selon l'une quelconque des revendications précédentes, dans lequel le premier non-tissé est une zone d'abattage non tissée (44) et le second non-tissé est une couverture externe 'enveloppe de couche (25) de l'article absorbant.

6. Article absorbant selon l'une quelconque des revendications 1 à 4, dans lequel le premier matériau non tissé est une couche supérieure d'enveloppe d'âme (16) et le second matériau non tissé est une couche inférieure d'enveloppe d'âme (16').

7. Article absorbant selon la revendication précédente, dans lequel la zone de liaison fait partie de la liaison centrale périmétrique (280', 282', 284', 286').

8. Article absorbant selon la revendication 7, dans lequel la zone de liaison est une zone de liaison de canal central (27).

9. Article absorbant selon l'une quelconque des revendications précédentes, dans lequel la viscosité de la composition de charge polymère à 170 °C va d'environ 1 000 mPa·s à environ 5 000 mPa.s, telle que mesurée selon le procédé d'essai de viscosité tel que décrit ici.

10. Article absorbant selon l'une quelconque des revendications précédentes, dans lequel le premier et/ou le second matériau non tissé comprennent des fibres naturelles ou recyclées.

11. Article absorbant selon l'une quelconque des revendications précédentes, dans lequel la zone de liaison a un temps de Force de pelage statique d'au moins 500 minutes par longueur liée de 10 mm, tel que mesuré selon le procédé de Test de Force de pelage statique décrit ici.

**12.** Conditionnement comprenant une pluralité d'articles absorbants selon l'une quelconque des revendications précédentes.

**13.** Premier matériau non tissé (100) et second matériau non tissé (110) ayant au moins une zone de liaison (120) entre le premier matériau non tissé et le second matériau non tissé, dans lesquels une composition de charge polymère (130) est disposée au sein de la zone de liaison, et dans lesquels la composition de charge polymère comprend :

- un ou plusieurs polymères choisis dans le groupe constitué d'homopolymères de polypropylène, de copolymères de propylène-éthylène, et de mélanges de ceux-ci ; et
- éventuellement de 0 % à moins de 5 % d'un agent collant, en poids de la composition de charge polymère ; et dans lesquels la composition de charge polymère a une ténacité d'au moins 25 MJ.m$^{-3}$, dans lesquels la ténacité est mesurée selon le procédé d'essai par extension décrit ici.

**14.** Procédé de liaison d'un premier matériau non tissé à un second matériau non tissé, comprenant l'étape consistant à appliquer une composition de charge polymère sur le premier matériau non tissé à une zone de liaison et lier le second matériau non tissé au premier matériau non tissé dans la zone de liaison, dans lequel la composition de charge polymère comprend :

- un ou plusieurs polymères choisis dans le groupe constitué d'homopolymères de polypropylène, de copolymères de propylène-éthylène, et de mélanges de ceux-ci ; et
- de 0 % à moins de 5 % d'un agent collant, en poids de la composition de charge polymère ;
dans lequel la composition de charge polymère a une ténacité d'au moins 25 MJ.m$^{-3}$, dans lequel la ténacité est mesurée selon le procédé d'essai par extension décrit ici.

**15.** Utilisation d'une composition de charge polymère pour augmenter la résistance au fluage de la zone de liaison entre un premier matériau non tissé et un second non-tissé dans un article absorbant (100) ; dans laquelle la composition de charge polymère (120) est disposée au sein de la zone de liaison, et dans laquelle la composition de charge polymère comprend :

- un ou plusieurs polymères choisis dans le groupe constitué d'homopolymères de polypropylène, de copolymères de propylène-éthylène, et de mélanges de ceux-ci ; et
- de 0 % à moins de 5 % d'un agent collant, en poids de la composition de charge polymère ;
dans laquelle la composition de charge polymère a une ténacité à 23 °C d'au moins 25 MJ.m$^{-3}$, dans laquelle la ténacité est mesurée selon le procédé d'essai par extension décrit ici.

**Fig. 1**

**Fig. 2**

**Fig. 3**

**Fig. 4**

**Fig. 5**

**Fig. 6**

Fig. 7

**Fig. 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160053149 A1, Clariant
- US 20160270987 A1
- US 20160270986 A1
- US 20170165130 A1
- US 20170165133 A1
- WO 201493323 A
- WO 2015183669 A, Bianchi
- WO 2015031225 A, Roe
- WO 2016133712 A, Ehmsperger
- US 5599335 A, Goldman
- EP 1447066 A, Busam
- WO 9511652 A, Tanzer
- US 20080312622 A1, Hundorf
- WO 2012052172 A, Van Malderen
- US 2006024433 A, Blessing
- US 20080312617 A
- US 20100051166 A1, Hundorf
- WO 2012170778 A, Rosati
- US 20120312491 A, Jackels
- US 7744576 B
- US 20110268932 A1
- US 20110319848 A1
- US 20110250413 A1
- US 20110250413 A, Hu
- US 20140072767 A1, Klaska

- WO 2012170798 A1, Jackels
- EP 2905000 A, Jackels
- EP 2905001 A, Armstrong-Ostle
- US 20140005020 A
- US 9421137 B
- US 6645190 B
- US 8747379 B
- US 8372052 B
- US 8361048 B
- US 6761711 B
- US 6817994 B
- US 8007485 B
- US 7862550 B
- US 6969377 B
- US 7497851 B
- US 6849067 B
- US 6893426 B
- US 6953452 B
- US 6840928 B
- US 8579876 B
- US 7682349 B
- US 7156833 B
- US 7201744 B
- US 5723546 A